# EUROPEAN PATENT APPLICATION

(11) **EP 4 790 713 A1**
(43) Date of publication of application: **12.08.2026**
(21) Application number: 24874583.8
(22) Date of filing: 01.10.2024
(51) Int. Cl.: G16B 30/00, C07K 16/00, C12N 15/13, G01N 33/48, G01N 33/53

(54) **ANTIBODY SELECTION METHOD, COMPUTER PROGRAM, AND INFORMATION PROCESSING DEVICE**

(30) Priority: 04.10.2023 JP 2023173199
(71) Applicant: The University of Osaka, Osaka 565-0871 (JP)
(72) Inventor: STANDLEY, Daron Michaelangelo, Suita-shi, Osaka 565-0871 (JP); ISMANTO, Hendra Saputra, Suita-shi, Osaka 565-0871 (JP); SAPUTRI, Dianita Susilo, Suita-shi, Osaka 565-0871 (JP)
(74) Representative: Bandpay & Greuter
(86) International application number: PCT/JP2024/035039
(87) International publication number: WO 2025/074981

(57) **Abstract**

An antibody selection method, a computer program, and an information processing apparatus are provided.

An antibody selection method executed by a computer, the method includes acquiring sequence data of a plurality of antibodies including antibodies with unknown antigen specificity, clustering acquired sequence data of the plurality of antibodies from a viewpoint of similarity of paratope pseudo sequences, determining clusters with specificity for a specific antigen by using a subset of antibodies within each cluster, and selecting antibodies with specificity for the specific antigen by inferring that antibodies outside the subset within the determined cluster have the same antigen specificity as that of antibodies within the subset.

## Description

### [Technical Field]

The present disclosure relates to an antibody selection method, a computer program, and an information processing apparatus.

### [Background Art]

An adaptive immune system includes T cell receptors and B cell receptors as molecules that specifically recognize a wide variety of pathogens. Since organisms cannot predict pathogens that invade from the outside world, they respond to a wide variety of pathogens by maintaining the diversity of T cell receptors and B cell receptors. The T cell receptor and the B cell receptor are composed of combinations of a plurality of gene regions, and for example, it is estimated that, in humans, there are 10¹⁵ to 10²⁰ types of T cell receptors and 10¹⁶ to 10¹⁸ types of B cell receptors.

As a method for determining a profile of recombinant DNA sequences in T cells and B cells, Patent Literature 1 discloses a method of collecting a sample derived from a subject including T cells and B cells, spatially isolating individual molecules of genomic DNA from the cells, determining the sequences of the spatially isolated individual molecules, and creating a profile of recombinant DNA sequences.

### [Prior Art Document]

### [Patent Document]

[Patent Document 1] Japanese Translation of PCT International Application Publication No. 2012-508011

### [Summary of Invention]

### [Problems to be Solved by Invention]

The method of Patent Literature 1, in which the profile of recombinant DNA sequences is created for individual T cells and B cells, has limitations in selecting an antibody having specificity for a specific antigen, from various antibodies.

In one aspect, an object is to provide an antibody selection method, a computer program, and an information processing apparatus capable of selecting an antibody having specificity for a specific antigen.

### [Means for Solving Problems]

An antibody selection method of the present disclosure executes: acquiring sequence data of a plurality of antibodies including antibodies with unknown antigen specificity, clustering acquired sequence data of the plurality of antibodies from a viewpoint of similarity of paratope pseudo sequences, determining clusters with specificity for a specific antigen by using a subset of antibodies within each cluster, and selecting antibodies with specificity for the specific antigen by inferring that antibodies outside the subset within the determined cluster have the same antigen specificity as that of antibodies within the subset.

### [Effects of Invention]

In one aspect, an antibody having specificity for a specific antigen can be selected.

### [Brief Description of Drawings]

Fig. 1 is an explanatory diagram illustrating an outline of an antibody selection method according to a first embodiment.
Fig. 2 is a block diagram illustrating an internal configuration of an information processing apparatus.
Fig. 3 is an explanatory diagram illustrating a clustering method according to the present embodiment.
Fig. 4 is an explanatory diagram illustrating effectiveness of clustering.
Fig. 5 is an explanatory diagram illustrating a clustering result of antibodies including known antibodies and novel antibodies.
Fig. 6 is an explanatory diagram illustrating evaluation results using cluster representatives.
Fig. 7 is a flowchart illustrating a procedure of processing executed by an information processing apparatus according to the first embodiment.
Fig. 8 is an explanatory diagram illustrating an outline of an antibody selection method according to a second embodiment.
Fig. 9 is an explanatory diagram illustrating a process of collecting BCR data.
Fig. 10 is a diagram showing a result of clustering using antigen probes.
Fig. 11 is an explanatory diagram illustrating an evaluation method.
Fig. 12 is an explanatory diagram illustrating evaluation results of antigen specificity.
Fig. 13 is a flowchart illustrating a procedure of processing executed by an information processing apparatus according to the second embodiment.

### [Mode for Carrying out Invention]

Hereinafter, the present invention will be described in detail with reference to the drawings illustrating embodiments thereof.

### (First embodiment)

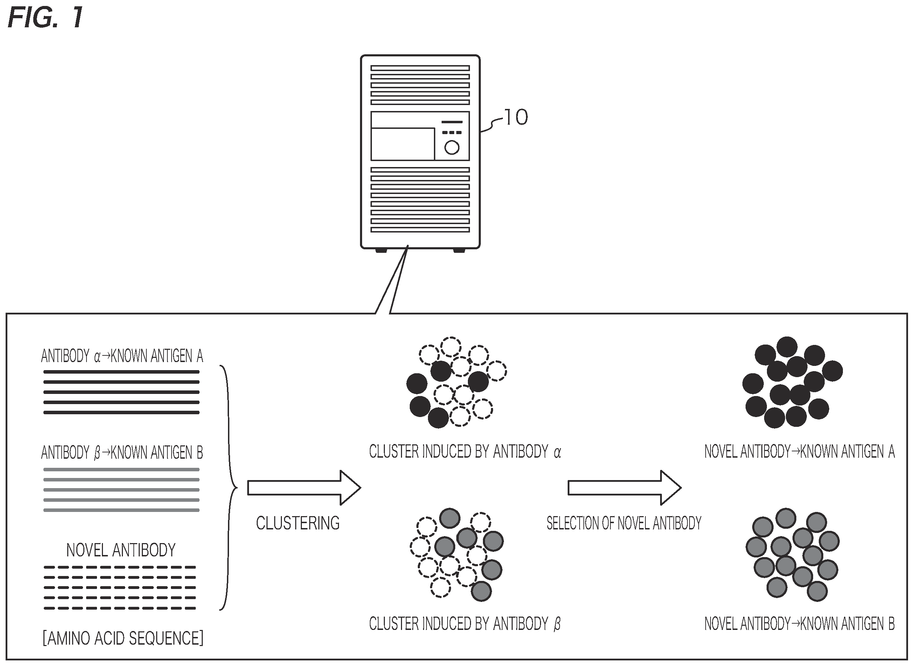

Fig. 1 is an explanatory diagram illustrating an outline of an antibody selection method according to a first embodiment. The present disclosure focuses on a method for selecting novel antibodies with specificity for a specific antigen. Here, the specificity (also referred to as antigen specificity) refers to the property of an antibody to react only with a specific antigen. An information processing apparatus 10 according to the present first embodiment acquires sequence data of amino acid sequences of an antibody having known antigen specificity and an antibody having unknown antigen specificity (novel antibody), and clusters the acquired sequence data to select a novel antibody having antigen specificity for a specific antigen.

In Fig. 1, sequence data of an antibody α having specificity for a known antigen A is schematically shown by a black solid line, sequence data of an antibody β having specificity for a known antigen B is schematically shown by a gray solid line, and sequence data of a novel antibody is schematically shown by a broken line. In addition, the antibody α is schematically shown by a black circle, the antibody β is schematically shown by a gray circle, and the novel antibody is schematically shown by a circle surrounded by a broken line. For example, the information processing apparatus 10 acquires sequence data of these antibodies, and clusters the sequence data of each antibody including the antibody α, the antibody β, and the novel antibody by utilizing the sequence data of each antibody.

As a result of clustering, the sequence data of one or more novel antibodies may be included in a cluster obtained by clustering mainly based on the sequence data of the antibody α. In addition, the cluster obtained by clustering mainly based on the sequence data of the antibody β may include sequence data of one or more other novel antibodies. At this time, the information processing apparatus 10 infers that the novel antibody included in the cluster of antibodies α has specificity for the known antigen A, and infers that the novel antibody included in the cluster of antibodies β has specificity for the known antigen B. In this manner, the information processing apparatus 10 can select novel antibodies with specificity for the known antigen A and the known antigen B.

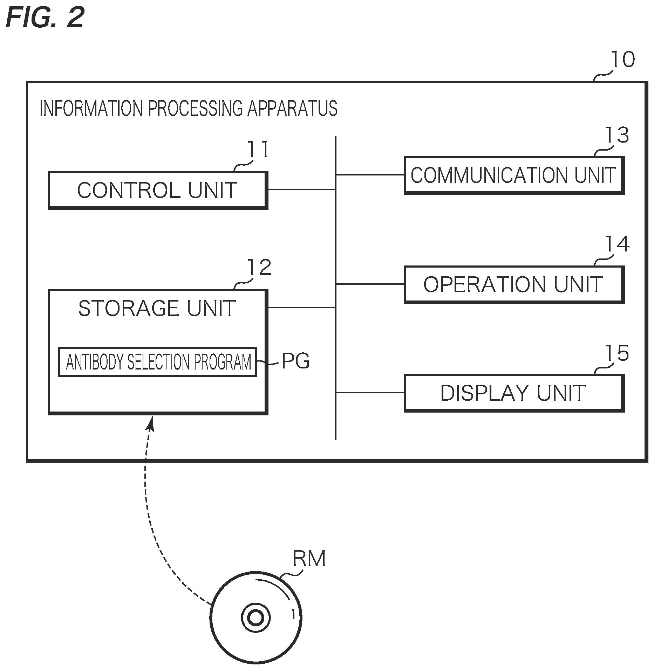

Fig. 2 is a block diagram illustrating an internal configuration of the information processing apparatus 10. The information processing apparatus 10 is a dedicated or general-purpose computer, and includes a control unit 11, a storage unit 12, a communication unit 13, an operation unit 14, and a display unit 15.

The control unit 11 includes, for example, a central processing unit (CPU), a read only memory (ROM), and a random access memory (RAM). The ROM included in the control unit 11 stores a control program and the like for controlling an operation of each unit of hardware included in the information processing device 10. The CPU in the control unit 11 reads and executes a control program stored in the ROM and a computer program to be described later stored in the storage unit 12 to control the operation of each unit of the hardware, thereby causing the entire apparatus to function as the information processing apparatus 10 of the present disclosure. The RAM included in the control unit 11 temporarily stores data utilized during execution of computation and control.

In the embodiment, the control unit 11 includes the CPU, the ROM, and the RAM, but the configuration of the control unit 11 is not limited to the above configuration. The control unit 11 may be, for example, one or more control circuits or arithmetic circuits including a graphics processing unit (GPU), a field programmable gate array (FPGA), a digital signal processor (DSP), a quantum processor, a volatile or nonvolatile memory, or the like. In addition, the control unit 11 may have functions such as a clock that outputs date and time information, a timer that measures an elapsed time from when a measurement start instruction is given to when a measurement end instruction is given, and a counter that counts numbers.

The storage unit 12 includes a storage device such as a hard disk drive (HDD) or a solid state drive (SSD). The storage unit 12 stores various computer programs executed by the control unit 11 and various data utilized by the control unit 11.

A computer program stored in a storage unit 12 includes an antibody selection program PG for causing a computer to execute processing including: acquiring sequence data of a plurality of antibodies including antibodies with unknown antigen specificity; clustering acquired sequence data from a viewpoint of similarity of paratope pseudo sequences; determining clusters with specificity for a specific antigen by using a subset of antibodies within each cluster; and selecting antibodies with specificity for the specific antigen by inferring that antibodies outside the subset within the determined cluster have the same antigen specificity as that of antibodies within the subset.

Each of the computer programs may be a single computer program or the computer programs may be a program group including a plurality of computer programs. In addition, the computer program may partially use an existing library. Each of the computer programs may be executed by a single computer, or the computer programs may be executed by a plurality of computers in cooperation with each other.

The computer program may be provided by a non-transitory recording medium RM (program product) in which the computer program is readably recorded. The recording medium RM is a portable memory such as a CD-ROM, a USB memory, or a secure digital (SD) card. The control unit 11 reads various computer programs from the recording medium RM by using a reading device (not illustrated), and stores the read various computer programs in the storage unit 12. In addition, the computer program may be provided by communication. The control unit 11 acquires a computer program by communication via the communication unit 13, and stores the acquired computer program in the storage unit 12.

The communication unit 13 includes a communication interface for transmitting and receiving various data to and from an external device. As the communication interface of the communication unit 13, a communication interface conforming to a communication standard such as WiFi (registered trademark), a local area network (LAN), Bluetooth (registered trademark), ZigBee (registered trademark), 3G, 4G, 5G, or long term evolution (LTE) can be used. In a case where data to be transmitted is input from the control unit 11, the communication unit 13 transmits data to a destination external device, and in a case where data transmitted from the external device is received, the communication unit 13 outputs the received data to the control unit 11. In the present embodiment, an example of the external device is a terminal device used by a medical worker such as a doctor or a subject.

The operation unit 14 includes operation devices such as a touch panel, a keyboard, and a switch, and receives various operations and settings by a user or the like. The control unit 11 performs appropriate control on the basis of various operation information given from the operation unit 14, and stores setting information in the storage unit 12 as necessary.

The display unit 15 includes a display device such as a liquid crystal display or an organic electro-luminescence (EL) display, and displays information to be presented, in response to an instruction from the control unit 11.

In the present embodiment, the information processing apparatus 10 may be a single computer or a computer system including a plurality of computers, peripheral devices, and the like. In addition, the information processing apparatus 10 may be a virtual machine having virtualized entity, or may be a cloud.

Hereinafter, details of processing executed by the information processing apparatus 10 will be described.

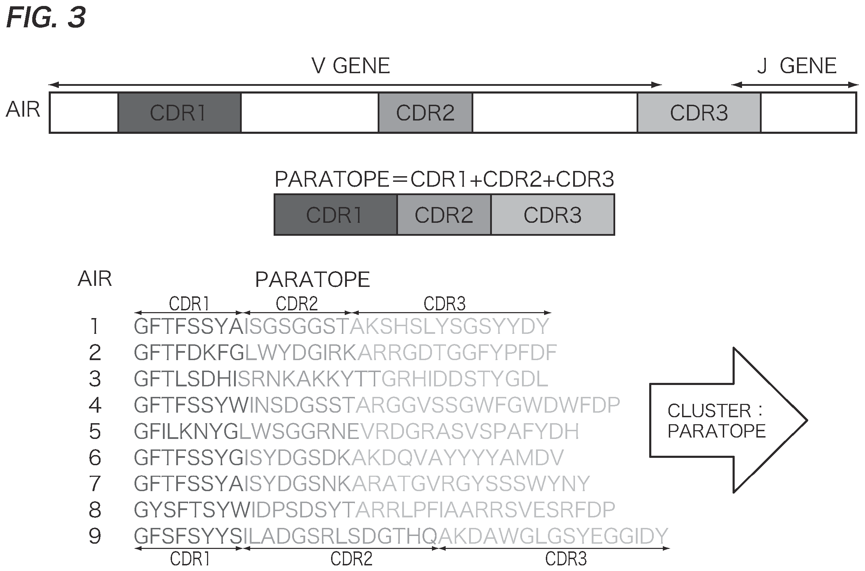

As described above, the information processing apparatus 10 clusters antibodies on the basis of sequence data of a known antibody and a novel antibody. Fig. 3 is an explanatory diagram illustrating a clustering method according to the present embodiment. Various antibodies consist of amino acid sequences and are described by a V gene name, a J gene name, and the position of the complementarity-determining region within the amino acid sequences. The complementarity-determining region is also referred to as a CDR (complementarity-determining region). The CDR includes three regions (CDR1, CDR2, CDR3). In the present embodiment, three segments of CDR1, CDR2, and CDR3 were pseudo-concatenated to construct one pseudo sequence, and sequence data of antibodies were clustered from the viewpoint of similarity of the pseudo sequence (hereinafter, referred to as a paratope pseudo sequence). Hereinafter, this clustering method is also referred to as a CDR clustering method. Note that an AIR in the drawing represents an adaptive immune receptor (AIR).

The clustering based on paratope pseudo sequences was performed by setting a sequence identity threshold and a coverage threshold to appropriate values and using an MMseqs2 search method. Alignment of paratope pseudo sequences with fixed sequence identity and coverage is statistically more significant than CDR3 alignment under identical criteria, due to the long sequence length.

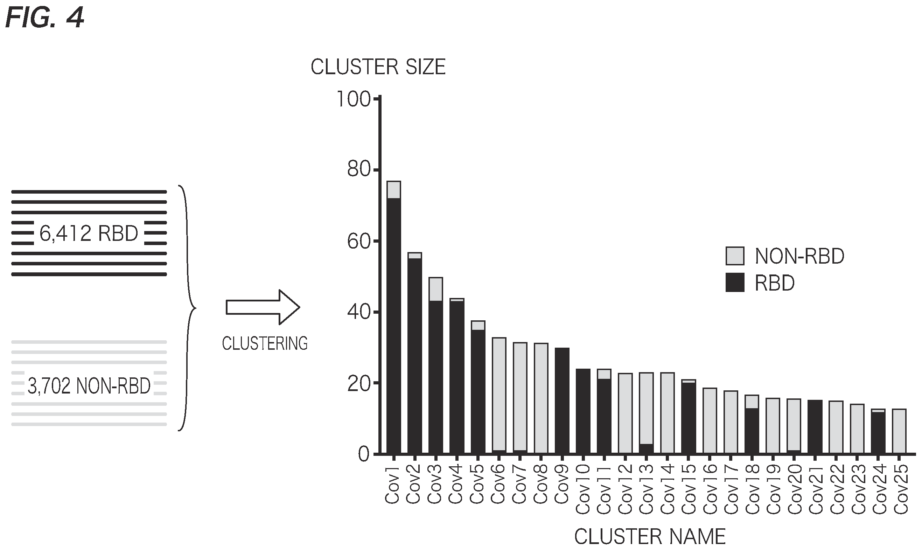

Fig. 4 is an explanatory diagram illustrating effectiveness of clustering. In order to validate the effectiveness of clustering utilizing paratope pseudo sequences, a publicly available BCR database (CovAbDab) containing a vast collection of BCRs (B cell receptors) with known specificity for coronavirus antigens was utilized. Non-human antibodies were excluded from the data sets registered in the database, and attention was paid to the sequence data of 6412 antibodies recognizing the receptor-binding domain (RBD) of SARS-CoV-2 spike proteins (hereinafter, denoted as RBD) and sequence data of 3702 antibodies targeting other regions of the spike proteins (hereinafter, denoted as non-RBD).

Paratope pseudo sequences were created from the BCR data of each antibody acquired from the BCR database, and then the sequence identity threshold and the coverage threshold were set to appropriate values, and clustering was performed from the viewpoint of similarity of the paratope pseudo sequences. Among 1074 non-singleton clusters that were clustered, 25 clusters were extracted in descending order of cluster size, and proportions of RBD and non-RBD in each cluster were shown in the graph of Fig. 4. In the graph of Fig. 4, a horizontal axis represents a cluster name, and a vertical axis represents a cluster size. According to the graph of Fig. 4, it can be seen that among the 25 clusters, the clusters of Cov1 to Cov5, Cov9 to Cov11, Cov15, Cov18, Cov21, and Cov24 are mainly based on RBD, and the other clusters are mainly based on non-RBD.

As a result of performing clustering with varying the sequence identity threshold and the coverage threshold, average cluster purity was found to be sensitive to both the sequence identity threshold and the coverage threshold. Here, the cluster purity is an index indicating how much sequence data within the cluster matches a specific annotation (in this case, either RBD or non-RBD). For example, the cluster purity of the cluster with the highest frequency of RBD (such as Cov1) is calculated by RBD/(RBD + non-RBD) × 100 (%), and the cluster purity of the cluster with the highest frequency of non-RBD (such as Cov6) is calculated by non-RBD/(RBD + non-RBD) × 100 (%).

The threshold for sequence identity was adjusted to achieve cluster purity of 95% or greater for each cluster. The coverage threshold was set at 90% to reduce the likelihood of sequences straddling CDR boundaries. For example, when the sequence identity threshold was set to 80% and the coverage threshold was set to 90%, the cluster purity of the top 25 clusters shown in the graph of Fig. 4 was 95.3%. The sequence identity threshold and the coverage threshold are not limited to the above values, and may be appropriately set such that the cluster purity of each cluster is higher than the set value.

In the alignment of the CDRH sequences (where CDRH represents the complementarity-determining regions of the H chain) of the top three largest clusters, it was found that while CDRH3 showed the highest variation, variation also occurred in CDRH1 and CDRH2. The average sequence identity of a pair of CDRH3 within a cluster is about 10% lower than the lower limit of a typical clonotype (80%), suggesting that CDR clustering is capable of grouping antibodies from clonal populations that share paratope antigen specificity.

Next, an application example to novel antibodies will be described.

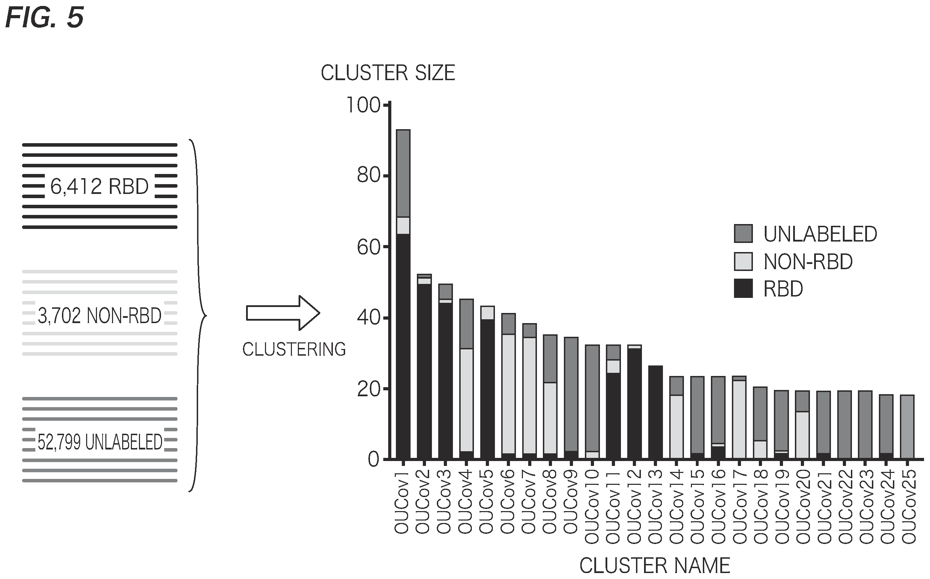

Fig. 5 is an explanatory diagram illustrating a clustering result of antibodies including known antibodies and novel antibodies. For the sequence data of known antibodies, as described above, non-human antibodies were excluded from the data sets registered in the BCR database (CovAbDab), and sequence data of 6412 antibodies recognizing the receptor-binding domain of SARS-CoV-2 spike proteins (RBD) and sequence data of 3702 antibodies targeting other regions of the spike proteins (non-RBD) were extracted and used. As the sequence data of the novel antibodies, 52799 pieces of BCR sequence data obtained from COVID-19 patients were used. Since it is unknown whether the novel antibodies are RBD antibodies or non-RBD antibodies, and any one thereof cannot be labeled, the novel antibody sequence data is hereinafter also referred to as unlabeled sequence data (or unlabeled).

As in the case of Fig. 4, paratope pseudo sequences were created from sequence data of antibodies including RBD, non-RBD, and unlabeled, and then the sequence identity threshold was set to 80% and the coverage threshold was set to 90%, and clustering was performed from the viewpoint of similarity of the paratope pseudo sequences. Out of 52799 pieces of unlabeled sequence data from COVID-19 patients, 1863 pieces were clustered with sequence data of known antibodies from the BCR database, resulting in a total of 602 clusters. Among the 1863 clusters that were clustered, 25 clusters were extracted in descending order of cluster size, and proportions of RBD, non-RBD, and unlabeled in each cluster were shown in the graph of Fig. 5. In the graph of Fig. 5, a horizontal axis represents a cluster name, and a vertical axis represents a cluster size. Referring to the graph of Fig. 5, it can be seen that unlabeled sequence data is included in OUCov1 to OUCov3, OUCov11, and the like, which are RBD-based clusters, and other unlabeled sequence data is included in OUCov4, OUCov6 to OUCov8, OUCov14, OUCov17, OUCov20, and the like, which are non-RBD-based clusters. The results of coclustering based on labeled sequence data (RBD and non-RBD) and unlabeled sequence data suggest that a public antibody response after SARS-CoV-2 infection can be detected by CDR clustering.

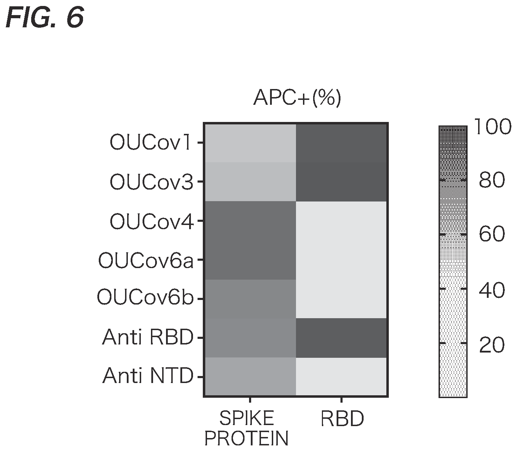

Fig. 6 is an explanatory diagram illustrating evaluation results using cluster representatives. A total of five unlabeled BCRs were picked up from OUCov1 and 3, which are RBD-based clusters, and OUCov4 and 6, which are non-RBD-based clusters, and antibodies were expressed from these five BCRs, respectively. Hereinafter, an antibody expressed from an unlabeled BCR is also referred to as an unlabeled antibody. These unlabeled antibodies were used as cluster representatives of respective clusters to evaluate specificity for full-length SARS-Cov-2 spike protein and RBD-expressing Expi293F cells.

Enzyme-Linked Immuno Sorbent Assay (ELISA) was used for evaluation. ELISA is a type of enzyme immunoassay that detects or quantifies target molecules by using antibodies. The evaluation results are shown in Fig. 6. A numerical value (0 to 100%) in Fig. 6 represents the proportion of the population of spike protein or RBD positively bound to the unlabeled antibodies. The evaluation results indicate that while all of the five unlabeled antibodies as cluster representatives bound to the spike protein, only the unlabeled antibodies expressed from the clusters of OUCov1 and 3 bound to the RBD, and the unlabeled antibodies expressed from the clusters of OUCov4 and 6 did not bind to the RBD. This indicates that the label assignment predicted from the graph of Fig. 5 is correct.

Note that from the CDRH alignment among the evaluated clusters, it was confirmed that the sequences of CDRH1 and CDRH2 were highly conserved but not completely conserved, and CDRH3 had a larger variation.

From the above findings, it was shown that CDR clustering is effective for assigning unlabeled BCRs to target antigens by using a limited data set of labeled antibodies (subset within each cluster).

Hereinafter, an operation of the information processing apparatus 10 will be described.

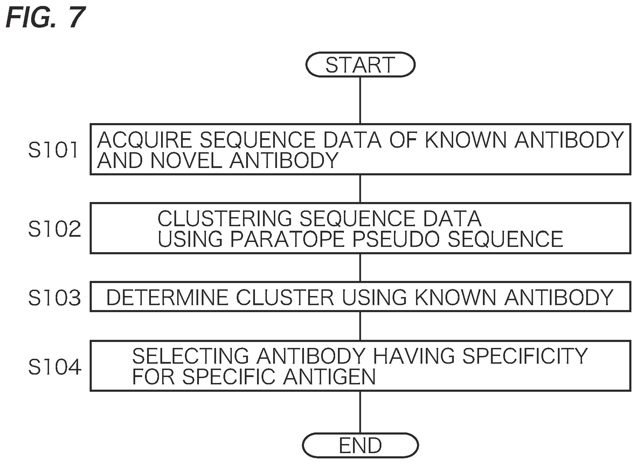

Fig. 7 is a flowchart illustrating a procedure of processing executed by the information processing apparatus 10 according to the first embodiment. The control unit 11 of the information processing apparatus 10 performs the following processing by reading and executing an antibody selection program PG from the storage unit 12.

The control unit 11 acquires sequence data of known antibodies and novel antibodies (step S101). The known antibodies are antibodies known to have specificity for a target antigen. In the above-described example, the antibodies recognizing RBD correspond to the known antibodies. In a case where there is a public BCR database such as CovAbDab, the control unit 11 can acquire sequence data of known antibodies by accessing the BCR database through the communication unit 13. The target antigen is not limited to SARS-CoV-2, and any antigen can be targeted. In addition, the target antigen does not need to be one type, and may be a plurality of types. When a plurality of types of antigens are targeted, it is sufficient to acquire sequence data of known antibodies each having specificity. The novel antibodies are antibodies with unknown antigen specificity. In the example described above, the unlabeled antibodies correspond to the novel antibodies. The sequence data of the novel antibodies is identified by using a known analysis method such as fluorescence-activated cell sorting (FACS) or single-cell immune profiling. In step S101, it is sufficient to acquire the sequence data of the novel antibodies identified by using the known analysis method. Labels for identifying the type of antigen are assigned to the sequence data of the known antibodies, and labels indicating that the antigen is unknown are assigned to the sequence data of the novel antibodies.

The control unit 11 clusters the sequence data of the known antibodies and the novel antibodies acquired in step S101 (step S102). On the basis of the acquired sequence data of each antibody, the control unit 11 creates a paratope pseudo sequence obtained by pseudo-concatenating the segments of CDR1, CDR2, and CDR3, and clusters the sequence data from the viewpoint of similarity of the paratope pseudo sequence. For example, the control unit 11 can cluster the sequence data by setting the sequence identity threshold and the coverage threshold to appropriate values and using the MMseqs2 search method.

The control unit 11 determines known antibody-based clusters on the basis of the result of clustering (step S103). The control unit 11 can determine how much sequence data of known antibodies is included in each cluster and how much sequence data of novel antibodies is included in each cluster, by referring to the label attached to each sequence data. The control unit 11 determines known antibody-based clusters by calculating a proportion (cluster purity) of the known antibodies included in each cluster.

The control unit 11 selects novel antibodies with specificity for the target antigen, on the basis of the result of clustering in step S102 and the determination result in step S103 (step S104). The control unit 11 determines whether or not novel antibodies are included in the cluster of known antibodies, by referring to the label attached to the sequence data. When it is determined that novel antibodies are included in the cluster of known antibodies, the antigen specificity of the novel antibodies is inferred to be the same as the antigen specificity of the known antibodies in the same cluster. According to the above procedure, the control unit 11 can select novel antibodies with specificity for the target antigen.

As described above, in the first embodiment, by clustering sequence data of antibodies with known antigen specificity and sequence data of novel antibodies with unknown antigen specificity and identifying the antigen specificity of a subset (known antigen) in the cluster, it is possible to select novel antibodies with specificity for a specific antigen.

### (Second embodiment)

In the first embodiment, sequence data of antibodies with known antigen specificity is acquired in advance, and clustering is performed together with sequence data of antibodies with unknown antigen specificity. However, after only sequence data of antibodies with unknown antigen specificity is acquired and clustering is performed, the antigen specificity may be identified for a subset in the cluster.

In a second embodiment, a configuration for identifying antigen specificity for a subset in a cluster after clustering is described.

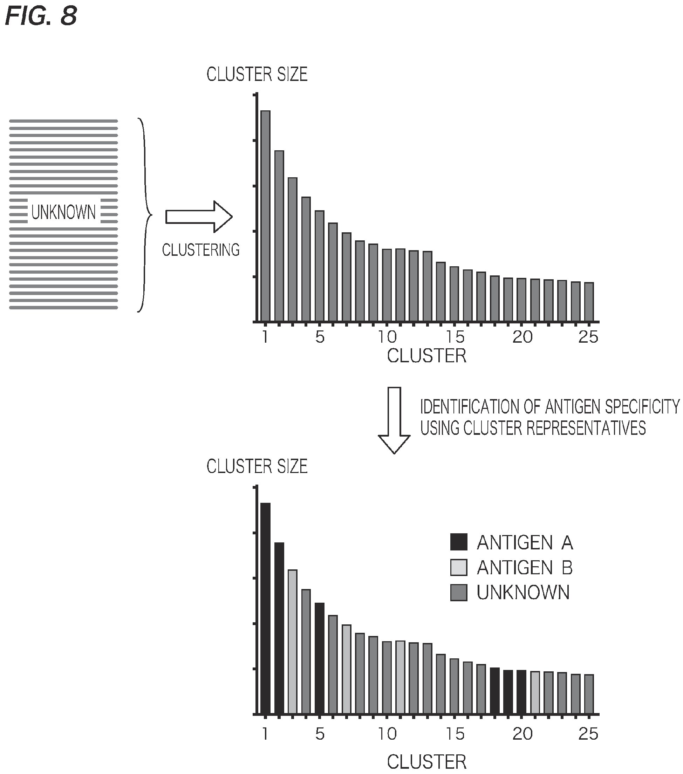

Fig. 8 is an explanatory diagram illustrating an outline of an antibody selection method according to the second embodiment. In the second embodiment, sequence data of antibodies with unknown antigen specificity is acquired, and the acquired clustering is performed from the viewpoint of similarity of paratope pseudo sequences. The clustering method is similar to that of the first embodiment, and for example, the sequence data can be clustered by setting the sequence identity threshold and the coverage threshold and using the MMseqs2 search method.

When 25 clusters are extracted in descending order of the cluster size among the clustered non-singleton clusters, and the cluster size of each cluster is shown in a graph, for example, a graph as shown in the upper right of Fig. 8 is obtained. At this time point, the antigen specificity of the antibodies identified by the sequence data in each cluster is unknown.

In the second embodiment, a small number (for example, 1 to 4) of sequence data is picked up from each cluster, and antigen specificity is identified for a small number of antibodies having the picked up sequence data. A set of a small number of sequence data (or antibodies) picked up from each cluster is also referred to as a cluster representative or a subset.

As described in the first embodiment, it can be inferred that each antibody having sequence data in the same cluster has the same antigen specificity. Therefore, by identifying the antigen specificity of the cluster representative antibodies (antibodies within the subset), it is possible to infer the antigen specificity of other antibodies (antibodies outside the subset) in the same cluster. For example, as a result of identifying antigen specificity by using the cluster representative of cluster 1, when it is found to have specificity for antigen A as shown in the lower right graph of Fig. 8, antibodies other than the cluster representative in cluster 1 can also be inferred to have specificity for antigen A. In addition, as a result of identifying antigen specificity by using the cluster representative of cluster 3, when it is found to have specificity for antigen B, antibodies other than the cluster representative in cluster 3 can also be inferred to have specificity for antigen B. The same applies to the antigen specificity of antibodies in another cluster.

Hereinafter, the usefulness of the CDR clustering method in annotation of antigens associated with another disease will be described. The DTP vaccine was chosen as an immune perturbation model since it has proven effectiveness in providing robust and broad protection against three highly lethal pathogens: diphtheria (D), tetanus (T), and pertussis (P). For this purpose, BCR data were collected from four healthy donors vaccinated with a trivalent DTP vaccine.

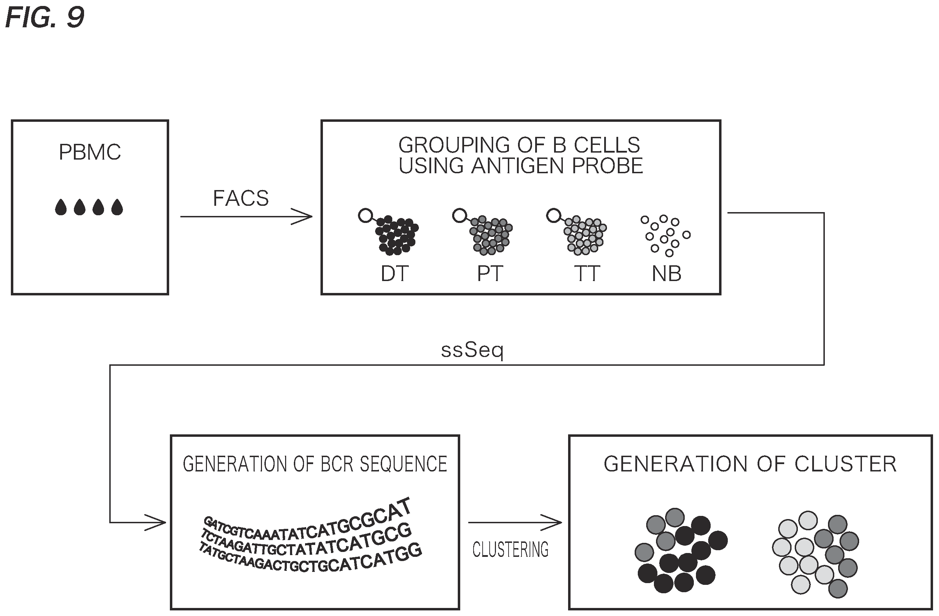

Fig. 9 is an explanatory diagram illustrating a process of collecting BCR data. In the present embodiment, peripheral blood mononuclear cells (PBMC) were collected from donors who received a DTP booster vaccination seven days after the booster administration. CD19+ CD27+ antigen-experienced B cells were selected from the collected peripheral blood mononuclear cells, and four different subgroups of B cells were further separated using fluorescently labeled DTP toxin/toxoid as an antigen probe. The four subgroups include three subgroups associated with diphtheria toxin (DT), tetanus toxin (TT), and pertussis toxin (PT), and a subgroup not associated with any toxin (NB).

Next, the four subgroups were then subjected to single-cell immune profiling and quality control procedures to acquire a total of 13847 BCR sequences consisting of both heavy (H) and light (L) chains.

As a result of performing both clonotype clustering and CDR clustering on the obtained BCR sequences, a total of 11636 clonotype clusters and 10802 CDR clusters were obtained. CDR clustering yielded more non-singleton clusters compared to clonotype clustering.

Among 4243 BCR sequences belonging to 1198 non-singleton clusters, 69 clusters in which members were assigned to a plurality of V genes, 46 clusters in which members were assigned to a plurality of J genes, and 1 cluster in which members were assigned to a plurality of V genes and J genes were found.

In total, 116 (9.68%) clusters were polyclonal, that is, consisted of a plurality of VDJ clonotypes. These results highlight a difference between clonotype clusters and CDR clusters, the latter demonstrating convergence from the different clonotypes into functionally similar paratopes in the antibody response after DTP vaccination. Nevertheless, clonotype clusters and CDR clusters showed comparable proportions, suggesting that the use of CDRs does not lead to excessive clustering.

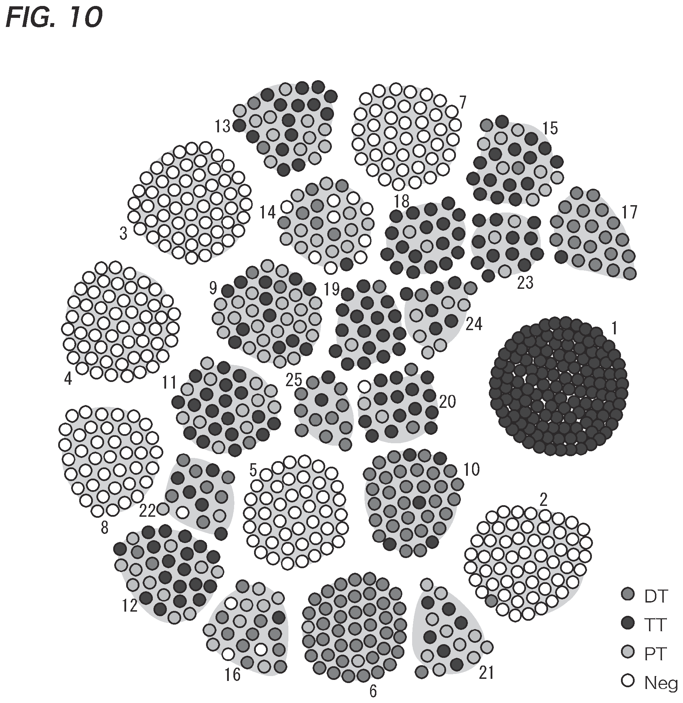

Fig. 10 is a diagram showing a result of clustering using antigen probes. In order to validate antigen specificity in clustering using antigen probes, 25 clusters were extracted in descending order of cluster size, and a label indicating the type of antigen was provisionally assigned to each BCR sequence on the basis of the antigen probe (DT, TT, PT, NB) bound in the process of cell selection. The label assigned to each BCR sequence includes DT, TT, PT, and Neg that does not correspond to any of them. The number 1 to 25 illustrated in the drawing indicates a cluster number.

Cluster 1 indicates a cluster containing only the BCR sequence bound to TT. In addition, clusters 3 to 5, 7, and 8 indicate clusters including only a BCR sequence that has not bound to any of DT, TT, and PT. On the other hand, other clusters include BCR sequences bound to a plurality of types of antigens. It was observed that the cluster purity of some clusters was low, and antibodies binding to different types of antigens were present in such clusters. The average cluster purity of the clusters using provisional labels was 82%, which was a value lower than 95% observed by COVID-19 analysis shown in the first embodiment.

Discrepancies in cluster purity between CDR clustering and provisional label assignment using antigen probes can be attributed to: (1) increased false positive rates caused by lenient gating strategies due to non-specific binding of fluorophores, streptavidin, and antigen purification tags; (2) sticky or polyreactive BCRs; and (3) a decrease in cluster purity when applied to raw BCR data that does not include valid antibodies.

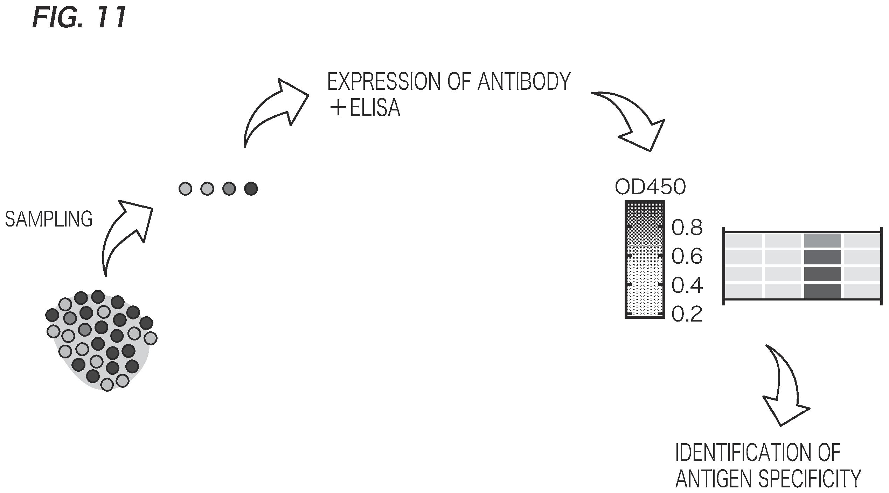

To address such a clustering result, a small number of BCR sequences were sampled from each cluster, antibodies were actually expressed, and the antigen specificity of each antibody was evaluated. Fig. 11 is an explanatory diagram illustrating an evaluation method. One to four BCR sequences with different provisional label assignments were sampled from each cluster. For example, since cluster 12 includes BCR sequences that bind to DT, TT, and PT, at least three BCR sequences including these are sampled as a subset. Antibodies based on a total of 52 BCR sequences selected from 25 clusters (human IgG1 monoclonal antibodies) were expressed. To evaluate the binding of these antibodies to each DTP toxoid (DT, TT, PT) and the SARS-CoV-2 spike protein as an irrelevant antigen, an indirect ELISA was performed.

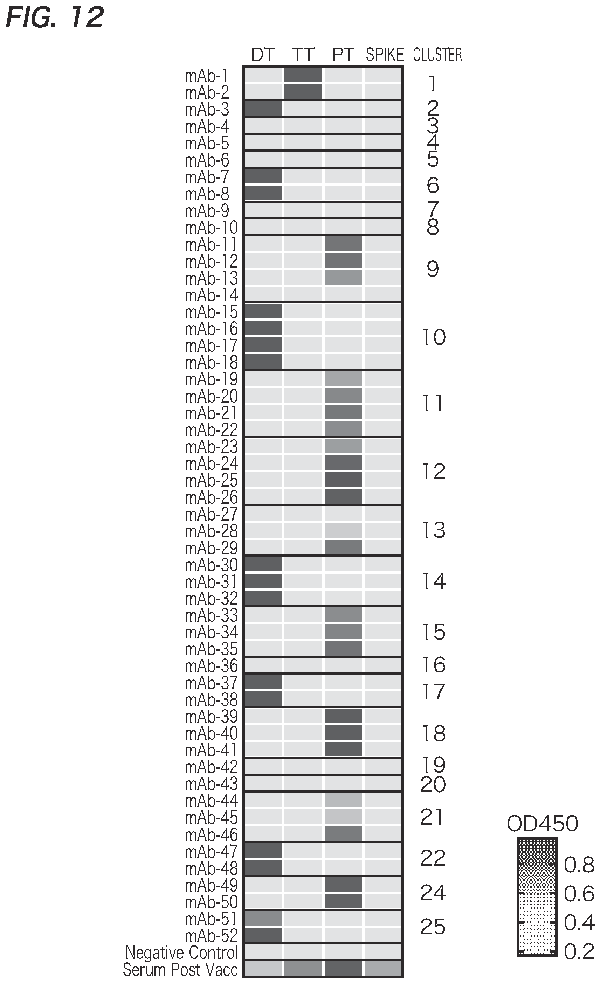

Fig. 12 is an explanatory diagram illustrating evaluation results of antigen specificity. Of the 52 antibodies derived from the top 25 clusters, 10 antibodies showed no reactivity to any of DT, TT, and PT, while 16 antibodies bound specifically to DT, 2 antibodies bound specifically to TT, and 24 antibodies bound specifically to PT. There were also antibodies determined to be negative in clusters 9 and 13, but none of the clusters contained members determined to be positive for a plurality of antigens. Therefore, within the range of sampling performed, the corrected cluster purity was found to be 96%. This is consistent with the COVID-19 evaluation described in the first embodiment.

On a basis of these evaluation results, 7, 1, and 8 clusters with specificity for DT, TT, and PT were identified, respectively. These results validated the antigen specificity of the CDR clusters and led to the selection of novel DTP-binding antibodies that may have neutralizing activity. By mapping all members of 25 large clusters, only 52 antibodies were evaluated to elucidate the specificity of over 800 unknown antibodies.

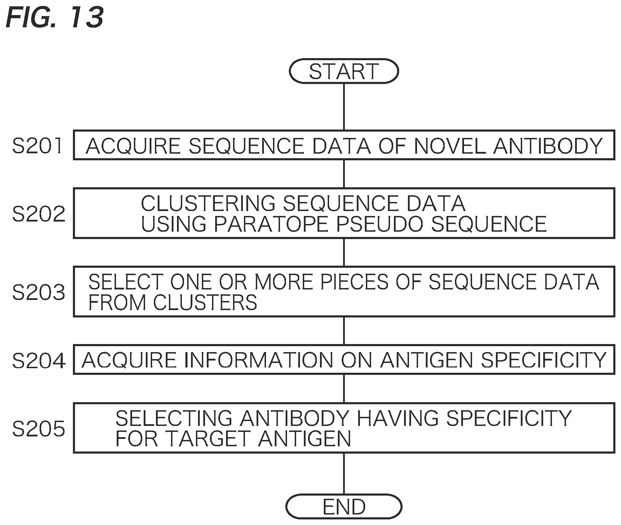

Fig. 13 is a flowchart illustrating a procedure of processing executed by the information processing apparatus 10 according to the second embodiment. The control unit 11 of the information processing apparatus 10 performs the following processing by reading and executing an antibody selection program PG from the storage unit 12.

The control unit 11 acquires sequence data of novel antibodies (step S201). In the present embodiment, sequence data provisionally labeled for a target antigen using antigen probes is acquired.

The control unit 11 clusters the sequence data of the novel antibodies acquired in step S201 (step S202). On the basis of the acquired sequence data of each antibody, the control unit 11 creates a paratope pseudo sequence obtained by pseudo-concatenating the segments of CDR1, CDR2, and CDR3, and clusters the sequence data from the viewpoint of similarity of the paratope pseudo sequence. For example, the control unit 11 can cluster the sequence data by setting the sequence identity threshold and the coverage threshold to appropriate values and using the MMseqs2 search method.

The control unit 11 selects one or more pieces of sequence data from a plurality of clusters obtained as a result of clustering (step S203). At this time, in a case where sequence data labeled with a plurality of types of antigens is included in the cluster, the control unit 11 is only required to select minimum sequence data labeled with different labels. In addition, when there is one type of label in the cluster, the control unit 11 is only required to select any one sequence data in the cluster.

For the sequence data selected by the control unit 11, the antibody identified by each sequence data is actually expressed, and the antigen specificity of each antibody is identified by using an existing method such as an ELISA method. Expression of antibodies and identification of antigen specificity are performed outside the information processing apparatus 10.

The control unit 11 acquires information on the antigen specificity identified for the antibody (step S204), and selects novel antibodies with specificity for the target antigen on the basis of the acquired information (step S205). The control unit 11 maps the antigen specificity of antibodies (antibodies in a subset) having sequence data selected in each cluster to other antibodies (antibodies outside the subset) in the cluster, thereby inferring the antigen specificity of other antibodies. According to the above procedure, the control unit 11 can select novel antibodies with specificity for the target antigen.

As described above, in the second embodiment, by clustering sequence data of novel antibodies with unknown antigen specificity and identifying the antigen specificity of a subset in the cluster, it is possible to select novel antibodies with specificity for the target antigen.

After selecting the novel antibodies with specificity for the target antigen, neutralizing antibodies against each antigen may be identified by observing the neutralizing activity of each antibody. An existing method is used for the identification method of the neutralizing antibodies. For example, a cytotoxicity assay may be performed to evaluate the inhibitory effect of DT-binding antibodies against VERO cell death induced by DT. In addition, an ELISA assay may be performed to examine the ability of TT binding antibodies to inhibit TT binding to the GT1b receptor. Furthermore, a CHO-K1 cell line that undergoes a morphological change when exposed to PT may be used to examine neutralization by PT-binding antibodies. Neutralizing antibodies can be identified by observing neutralizing activity utilizing these assays.

In the first and second embodiments, the clustering method utilizing paratope representations has been described, but when a decrease in sensitivity is acceptable, antibodies may be clustered utilizing clonotype representations. The clustering method using clonotype representations is a method of clustering antibodies from the viewpoint of similarity of CDR3 sequence, while having the same V gene and J gene.

The embodiment disclosed at present is construed as illustrative in all respects and not restrictive. The scope of the present invention is defined not by the above meaning but by the claims, and is intended to include all modifications within the meaning and scope equivalent to the claims.

### [Description of Reference Numerals]

- 10: information processing apparatus
- 11: control unit
- 12: storage unit
- 13: communication unit
- 14: operation unit
- 15: display unit
- PG: antibody selection program

## Claims

1. An antibody selection method executed by a computer, the method comprising:
acquiring sequence data of a plurality of antibodies including antibodies with unknown antigen specificity;
clustering acquired sequence data of the plurality of antibodies from a viewpoint of similarity of paratope pseudo sequences;
determining clusters with specificity for a specific antigen by using a subset of antibodies within each cluster; and
selecting antibodies with specificity for the specific antigen by inferring that antibodies outside the subset within the determined cluster have the same antigen specificity as that of antibodies within the subset.

2. The antibody selection method according to claim 1, wherein
the plurality of antibodies include an antibody with known specificity for the specific antigen, and
the method executed by the computer comprising:
inferring the antigen specificity of the antibodies outside the subset by using the antibody with known specificity in the subset.

3. The antibody selection method according to claim 1 executed by the computer, the method comprising:
identifying the antigen specificity of the antibodies within the subset; and
inferring the antigen specificity of the antibodies outside the subset on a basis of an identified result.

4. The antibody selection method according to claim 3 executed by the computer, the method comprising:
identifying the antigen specificity of the antibodies within the subset by expressing one or more antibodies sampled from the subset and acquiring results of antigen screening for the expressed antibodies.

5. The antibody selection method according to claim 4 executed by the computer, the method comprising:
classifying human peripheral blood mononuclear cells collected from a plurality of donors by using antigen probes, and assigning labels indicating type of antigens to B cells included in the human peripheral blood mononuclear cells on a basis of a classified result; and
selecting B cells assigned with different types of labels as B cells for producing the one or more antibodies.

6. The antibody selection method according to claim 4 executed by the computer, the method comprising:
identifying a cluster including antibodies having neutralizing activity against the antigen by observing the neutralizing activity of the antibodies for which the antigen specificity has been inferred.

7. The antibody selection method according to any one of claims 1 to 6, wherein
the paratope pseudo sequence is configured by pseudo-concatenating segments representing three complementarity-determining regions included in an antigen receptor.

8. A computer program causing a computer to execute processing of:
acquiring sequence data of a plurality of antibodies including antibodies with unknown antigen specificity;
clustering acquired sequence data of the plurality of antibodies from a viewpoint of similarity of paratope pseudo sequences;
determining clusters with specificity for a specific antigen by using a subset of antibodies within each cluster; and
selecting antibodies with specificity for the specific antigen by inferring that antibodies outside the subset within the determined cluster have the same antigen specificity as that of antibodies within the subset.

9. An information processing apparatus comprising:
at least one processor, wherein
the processor
acquires sequence data of a plurality of antibodies including antibodies with unknown antigen specificity;
clusters acquired sequence data of the plurality of antibodies from a viewpoint of similarity of paratope pseudo sequences;
determines clusters with specificity for a specific antigen by using a subset of antibodies within each cluster; and
selects antibodies with specificity for the specific antigen by inferring that antibodies outside the subset within the determined cluster have the same antigen specificity as that of antibodies within the subset.
